# EUROPEAN PATENT APPLICATION

(11) **EP 4 635 428 A1**
(43) Date of publication of application: **22.10.2025**
(21) Application number: 23943087.9
(22) Date of filing: 17.07.2023
(51) Int. Cl.: A61B 17/12, A61B 17/125

(54) **LIGATION DEVICE CAPABLE OF AUTOMATICALLY CUTTING LIGATURE WIRE**

(30) Priority: 30.06.2023 CN 202321699038 U
(71) Applicant: Medlead Medical Technology Co., Ltd., Shenzhen, Guangdong 518000 (CN)
(72) Inventor: HE, Guojun, Shenzhen, Guangdong 518000 (CN)
(74) Representative: Herrero & Asociados, S.L.
(86) International application number: PCT/CN2023/107660
(87) International publication number: WO 2025/000599

(57) **Abstract**

A ligation device capable of automatically cutting a ligature wire, the ligation device comprising a front handle (1), a rear-section handle (2), a catheter assembly, a snare (3) and a ligature wire (4), wherein one end of the catheter assembly is connected to the front handle (1), the snare (3) is arranged at the other end of the catheter assembly, and a slit is provided in the inner side of the snare (3); a ligature wire (4) passes through the front handle (1) and the catheter assembly; one end of the ligature wire (4) is connected to the rear-section handle (2), and the other end of the ligature wire (4) is a wire loop (41) that is retractable in the forward direction and stops in the reverse direction; the wire loop (41) is pre-assembled in the snare (3); a ligature wire cutting assembly is provided in the rear-section handle (2); and when the rear-section handle (2) is separated from the front handle (1) and is pulled backwards, the ligature wire (4) is pulled backwards, such that the wire loop (41) is pulled out of the slit to the inner side of the snare (3) and retracts, and when the ligature wire (4) is pulled backwards to reach a preset tension, the ligature wire cutting assembly automatically cuts the ligature wire (4). A ligation operation is achieved by means of pulling a rear-section handle (2) backwards, and automatic ligature wire cutting is performed at a preset node by means of the ligature wire cutting assembly during the operation process without needing to rely on the visual estimation and hand perception of an operating surgeon and thus the problem of manual operation failure or errors is avoided.

## Description

The present application claims priority of Chinese patent Application No. 202321699038.3, filed on June 30, 2023, the entire contents of which are hereby incorporated by reference in its entirety.

### TECHNICAL FIELD

The present disclosure relates to the technical field of medical devices, and in particular to an automatic ligature-cutting ligation device.

### BACKGROUND

A left atrial appendage (LAA) is a long and narrow tubular blind chamber. The LAA may have an irregular shape with multiple twists and turns. A size of the LAA may be similar to that of a thumb, and an opening size of the LAA may be in a range from 10 mm to 40 mm. The LAA may include abundant pectinate muscles and muscle trabeculae, such that blood flow in the LAA may be prone to vortex formation and reduced velocity, which may cause the LAA to be a common site for thrombus formation.

A patient with atrial fibrillation (AF) may have a significantly increased risk of stroke. Studies indicate that 60% of cardiogenic thrombi in patients with rheumatic heart disease complicated with the AF come from the LAA, and 91% of strokes caused by non-valvular AF are caused by thrombi in the LAA. Therefore, removing the LAA may help reduce left atrial thrombus and stroke events.

At present, clinical treatment methods include oral anticoagulant drugs, radiofrequency ablation, percutaneous left atrial appendage closure, and surgical LAA resection. However, regardless of the method, the clinical effect is not very satisfactory. At present, clinical treatment methods may include: oral anticoagulant drugs, radiofrequency ablation, percutaneous LAA closure, and surgical LAA resection. However, regardless of any one of the methods, a clinical effect is not very satisfactory.

Therefore, a LAA ligation is emerged. Accordingly, matching medical devices may also be developed.

In an existing LAA ligation device, such as Chinese patent Application No. 202110054297.X, when using LAA ligation device, a pre-formed ligature loop may be typically delivered to a target surgical area by utilizing a delivery tool, and an operator may pull the loop to tighten and complete the ligation by applying external hand force. During the above operation process, a judgment of whether the ligation is completed or firm mainly depends on a visual judgment and hand feeling of an operating doctor, which is prone to an operational error or deviation.

### SUMMARY OF THE DISCLOSURE

A purpose of the present disclosure is to provide an automatic ligature-cutting ligation device, so as to solve the problem in the related art that it is necessary to rely on the visual judgment and hand sensation of the operating doctor when performing a ligation operation by using an existing LAA ligation device , which is prone to an operational error or deviation.

To solve the above-mentioned technical problem, the purpose of the present disclosure may be achieved by the following technical solutions provided by some embodiments of the present disclosure. An automatic ligature-cutting ligation device may be provided by some embodiments of the present disclosure. The automatic ligature-cutting ligation device includes: a handle assembly, including a front handle and a rear handle detachably connected to each other; a catheter assembly, an end of the catheter assembly being connected to the front handle; a snare, disposed at another end of the snare, where a slit is defined in an inner surface of the snare; a ligature, penetrating the front handle and the catheter assembly, where an end of the ligature is connected to the rear handle, another end of the ligature has a loop capable of contracting in a forward direction and stopping in a reverse direction, and the loop is pre-mounted in the snare; and a ligature-cutting assembly, disposed in the rear handle; where in a case where the rear handle is separated from the front handle and pulled rearward, the ligature is driven to pull rearward, and the loop is driven to pull out of the snare from the slit to an inner side enclosed by the snare and contract in the forward direction; and in a case where the rear handle is separated from the front handle and pulled rearward until a preset pulling force is reached, the ligature-cutting assembly is driven to move and cut the ligature.

In some embodiments, an end of the front handle close to the rear handle is spirally interlocked with an end of the rear handle close to the front handle, and in a case where the rear handle rotates relative to the front handle, the rear handle is capable of being separated from or engaged with the front handle.

In some embodiments, the ligature-cutting assembly includes: a sliding member, slidably mounted in the rear handle and capable of sliding along a direction close to the front handle or away from the front handle, where an end of the ligature is connected to the sliding member; a connecting rod member, mounted in the rear handle, where an end of the connecting rod member is connected to the sliding member; an elastic member, an end of the elastic member being connected to an inner side of the rear handle, and another end of the elastic member being connected to the sliding member; and a ligature-cutting blade, connected to another end of the connecting rod member; where in a case where the rear handle is pulled rearward, the ligature is configured to drive the sliding member to slide forward, the elastic member is driven to stretch, and the connecting rod member is simultaneously driven to rotate, such that the ligature-cutting blade is driven to rotate towards the ligature; and in a case where the elastic member is stretched to the preset pulling force, the ligature is cut by the ligature-cutting blade.

In some embodiments, the connecting rod member includes a first connecting rod and a second connecting rod; an end of the first connecting rod is rotatably connected to the sliding member, and another end of the first connecting rod is rotatably connected to an end of the second connecting rod; and a rod arm of the second connecting rod is rotatably connected to an inner wall of the rear handle, and the ligature-cutting blade is disposed at another end of the second connecting rod.

In some embodiments, a strip-shaped opening is defined on the rear handle, the sliding member is arranged with an extension portion, and the extension portion passes through the strip-shaped opening and protrudes beyond the rear handle; and the extension portion is connected to another end of the elastic member.

In some embodiments, the elastic member includes one of a spiral spring and an elastic pull rope.

In some embodiments, a ligature-cutting base is disposed in the rear handle, the ligature-cutting blade is opposite to the ligature-cutting base, and the ligature is placed on the ligature-cutting base; and a V-shaped notch opposite to the ligature-cutting blade is defined on the ligature-cutting base.

In some embodiments, the catheter assembly includes a heat-shrinkable sleeve, a flexible catheter, and a connecting tube; an end of the flexible catheter is connected to a front end of the front handle; the heat-shrinkable sleeve is covered on a connection part between the flexible catheter and the front end of the front handle; and an end of the connecting tube is connected to another end of the flexible catheter, and another end of the connecting tube is connected to the snare.

In some embodiments, a bone positioning component inside the snare includes a nitinol memory alloy wire.

In some embodiments, a radiopaque tungsten wire is embedded in the snare, and a radiopaque coil spring is disposed in the connecting tube.

An automatic ligature-cutting ligation device may be provided in some embodiments of the present disclosure. The automatic ligature-cutting ligation device may include a front handle, a rear handle, a catheter assembly, a snare, a ligature, and a ligature-cutting assembly. An end of the catheter assembly may be connected to the front handle, and the snare may be disposed at another end of the snare. A slit may be defined in an inner surface of the snare. The ligature may penetrate the front handle and the catheter assembly. An end of the ligature may be connected to the rear handle, and another end of the ligature may be a loop capable of contracting in a forward direction and stopping in a reverse direction. The loop may be pre-mounted in the snare. The ligature-cutting assembly may be disposed in the rear handle. When the rear handle may be separated from the front handle and pulled rearward, the ligature may be driven to pull rearward, and the loop is driven to pull out of the snare from the slit to an inner side enclosed by the snare and contract. When the ligature is pulled rearward until a preset pulling force is reached, the ligature may be automatically cut by the ligature-cutting assembly. In the automatic ligature-cutting ligation device provided in the embodiments of the present disclosure, the ligation operation may be performed by pulling the rear handle rearward, and during the above operation process, the ligature-cutting assembly may automatically cut the ligature at a ligature-cutting node. Therefore, it is not necessary to rely on the visual judgment and hand feeling of the operating doctor, thereby reducing the occurrence of manual operation errors or deviations.

### BRIEF DESCRIPTION OF THE DRAWINGS

In order to more clearly describe the technical solutions in the embodiments of the present disclosure or the related art, the drawings that need to be used in the description of the embodiments or the related art will be briefly described in the following. Apparently, the drawings in the following description are only some embodiments of the present disclosure. For those skilled in the art, other drawings can be obtained based on these drawings without creative work.
FIG. 1 is a schematic structural view of an automatic ligature-cutting ligation device according to some embodiments of the present disclosure.
FIG. 2 is a schematic structural view of a handle assembly in a separated state according to some embodiments of the present disclosure.
FIG. 3 is an inner schematic structural view of a rear assembly according to some embodiments of the present disclosure.
FIG. 4 is an exploded structural schematic view of a front end of the automatic ligature-cutting ligation device according to some embodiments of the present disclosure.

Reference signs in drawings:
1, front handle;
2, rear handle; 21, sliding member; 211, extension portion; 22, first connecting rod; 23, second connecting rod; 24, elastic member; 25, ligature-cutting blade; 26, strip-shaped opening; 27, ligature-cutting base;
3, snare; 31, radiopaque tungsten wire;
4, ligature; 41, loop;
5, heat-shrinkable sleeve;
6, flexible catheter;
7, connection tube; 71, radiopaque coil spring.

### DETAILED DESCRIPTION

The technical solutions in the embodiments of the present disclosure will be clearly and completely described below with reference to the drawings in the embodiments of the present disclosure. It is apparent that the described embodiments are a part of the embodiments of the present disclosure rather, than all the embodiments. All other embodiments obtained by those of ordinary skill in the art without creative efforts based on the embodiments in the present disclosure shall fall within the protection scope of the present disclosure.

It should be understood that in a case where used in the description and the appended claims, terms "comprise/comprising" and "include/including" indicate the presence of the described features, integers, steps, operations, elements, and/or components, but do not exclude the presence or addition of one or more other features, integers, steps, operations, elements, components, and/or combinations thereof.

It should also be understood that terms used herein in the description of the embodiments of the present disclosure is for the purpose of describing particular embodiments only and is not intended to limit the embodiments of the present disclosure. As used in the description of the embodiments of the present disclosure and the appended claims, singular forms "a", "an" and "the" are intended to include plural forms unless the context clearly indicates otherwise.

It should be further understood that a term "and/or" used in the description of the present disclosure and the appended claims may be referred to any combinations and all possible combinations of one or more of the listed item and include the combinations.

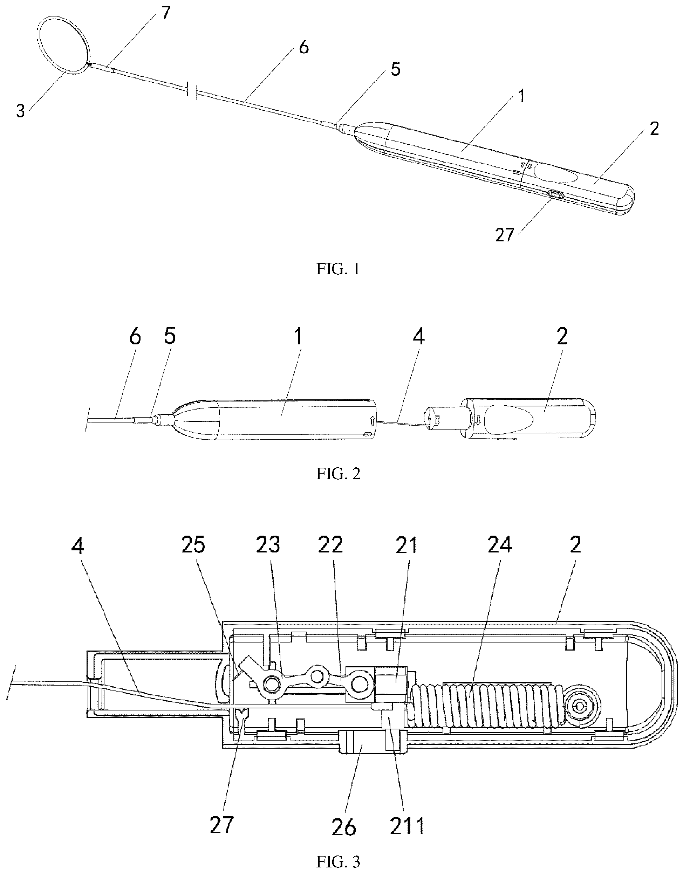

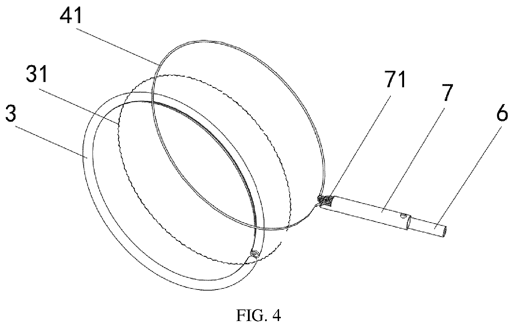

As shown in FIGS. 1-4, an automatic ligature-cutting ligation device may be provided by some embodiments of the present disclosure. The automatic ligature-cutting ligation device may include a handle assembly, a catheter assembly, a snare 3, and a ligature 4.

The handle assembly may include a front handle 1 and a rear handle 2 detachably connected to each other. An end of the catheter assembly may be connected to the front handle 1, and another end of the catheter assembly may be connected to the snare 3. A slit may be defined in/at an inner surface of the snare 3. The ligature 4 may penetrate the front handle 1 and the catheter assembly. An end of the ligature 4 may be connected to the rear handle 2. Another end of the ligature 4 may be a loop 41, and the loop 41 may be capable of contracting/tightening in a forward direction and stopping in a reverse direction. The loop 41 may be pre-mounted in the receiving channel.

When the rear handle 2 is separated from the front handle 1 and the rear handle 2 may be moved/pulled rearward, the ligature 4 may be driven to remove/pull rearward, such that the loop 41 may be driven to removed out/pulled out of the snare 3 from the slit to an inner side enclosed by the snare 3 and contract in the forward direction.

A ligature-cutting assembly may be disposed in the rear handle 2. When the rear handle 2 is separated from the front handle 1 and pulled rearward until a preset action force, such as a pulling force, is reached, the ligature-cutting assembly may be driven to move and cut the ligature 4.

An operation process of the automatic ligature-cutting ligation device provided by some embodiments of the present disclosure may be described as follows. An end of the catheter assembly connected to the snare 3 may be delivered into a heart through an outer sheath, and the snare 3 may be sleeved on the external tissue of inverted LAA inside a cardiac cavity. Subsequently, the rear handle 2 may be rotated to be separated from the front handle 1, and the rear handle 2 may be pulled rearward, such that the loop 41 may be pulled out of the snare 3 from the slit to the inner side enclosed by the snare 3, and the loop 41 may be contracted on a tissue to be ligated. As an action force, such as a pulling force, applied on the rear handle 2 is increased, the action force applied on the rear handle 2 may also reach the preset action force when the tissue ligation is completed. At this time, the ligature-cutting assembly may move under the pulling force and cut the ligature 4, such that an automatic ligature-cutting operation after a ligation operation may be completed.

In some embodiments, the pulling force required for completing the ligation operation may be measured, and a ligature-cutting node of the ligature-cutting assembly may be designed. In some embodiments, the pulling force required for completing the ligation may be generally about 30 N. When the ligature-cutting assembly is subjected to an action force of about 30 N, such as a pulling force of about 30 N, the ligature-cutting assembly may be driven to move to a position of the ligature 4, such that it may be possible to enable a blade of the ligature-cutting assembly to collide with the ligature 4 to cut the ligature 4. In this way, by pulling the second handle 2 rearward, it may be possible to complete the tissue ligation, and the ligature 4 may also be cut, such that during the operation process, it may be not necessary to rely on the visual judgment and hand feeling of the operating doctor, and thus it may be possible to reduce the errors or deviations caused by manual operation.

It should be noted that the preset pulling force of 30 N may be selected according to an actual application scenario in some embodiments of the present disclosure, and a specific value of the preset pulling force may be adaptively adjusted according to different situations.

In some embodiments, a rear end of the front handle 1 may be spirally interlocked with a front end of the rear handle 2. When the rear handle 2 rotates relative to the front handle 1, the rear handle 2 may be capable of being separated from or engaged with the front handle 1.

In some embodiments, mutually matching buckle structures may be respectively arranged on an inner wall of the rear end of the front handle 1 and an inner wall of the front end of the rear handle 2. By rotating the front handle 1 and the rear handle 2 relative to each other, the front handle 1 and the rear handle 2 may be relatively locked and fixed to implement the locking in a non-use state. In addition, the front handle 1 and the rear handle 2 may also be unlocked from each other and pulled after unlocking to implement the quick operation during use. Therefore, only actions of rotation and pulling may be needed during use, and the operation may be simple and convenient.

As shown in FIG. 3, in some embodiments, the ligature-cutting assembly may include a sliding member 21, a connecting rod member, an elastic member 24, and a ligature-cutting blade 25. The sliding member 21 may be slidably mounted in the rear handle 2 and capable of sliding forward/rearward. An end of the ligature 4 may be connected to the sliding member 21. The connecting rod member may be mounted in the rear handle 2. An end of the connecting rod member may be connected to the sliding member 21. The ligature-cutting blade 25 may be disposed at another end of the connecting rod member. An end of the elastic member 24 may be connected to the inner wall of the rear handle 2. Another end of the elastic member 24 may be connected to the sliding member 21.

When the rear handle 2 is pulled rearward, the ligature 4 may be configured to drive the sliding member 21 to slide forward, and the elastic member 24 may be driven to stretch. The ligature 4 may be configured to simultaneously drive the connecting rod member to rotate, so as to enable the ligature-cutting blade 25 to rotate toward the ligature 4. When the elastic member 24 is stretched to the preset pulling force, the ligature-cutting blade 25 may cut the ligature 4.

In some embodiments, when the rear handle 2 is pulled rearward 1, the ligature 4 may exert/generate a forward pulling force on the sliding member 21, such that the sliding member 21 may be driven to move forward. In this way, the connecting rod member may be driven to rotate through a forward movement of the sliding member 21, such that as the connecting rod member rotates, the ligature-cutting blade 25 may be driven to rotate toward the ligature 4. Therefore, when the pulling force applied on the rear handle 2 reaches the preset value, the ligature 4 may be cut by the ligature-cutting blade 25.

In some embodiments, the elastic member 24 may be a spiral spring, an elastic rope, or other structures with a rebound function.

In some embodiments, the connecting rod member may include a first connecting rod 22 and a second connecting rod 23. An end of the first connecting rod 22 may be rotatably connected to the sliding member 21, and another end of the first connecting rod 22 may be rotatably connected to an end of the second connecting rod 23. A rod arm of the second connecting rod 23 may be rotatably connected to the inner wall of the rear handle 2. The ligature-cutting blade 25 may be disposed at another end of the second connecting rod 23. When the automatic ligature-cutting ligation device is in use, the forward movement of the sliding member 21 may push the first connecting rod 22 and the second connecting rod 23 to rotate, so as to drive the ligature-cutting blade 25 to rotate toward the ligature 4. When the automatic ligature-cutting ligation device is not in use, the elastic member 24 may be capable of keeping the sliding member 21 in the reset state, so as to keep the ligature-cutting blade 25 away from the ligature 4, thereby prevent accidental cutting.

In some embodiments, a strip-shaped opening 26 may be defined on the rear handle 2. An extension portion 211 may be arranged on the sliding member 21. The extension portion 211 may pass through the strip-shaped opening 26 and may protrude beyond the rear handle. Another end of the elastic member 24 may be connected to the extension portion 211.

In some embodiments, a length direction of the strip-shaped opening 26 may be substantially parallel to a sliding direction of the sliding member 21. The extension portion 211 may be disposed in the strip-shaped opening 26, such that it may be possible to limit a sliding path of extension portion 211, thereby limiting a sliding path of the elastic member 24. In this way, on one hand, a sliding stroke of the sliding member 21 may be limited, such that it may be possible to limit the maximum pulling force achievable by pulling the rear handle 2 backward, thereby ensuring that excessive force does not occur. On the other hand, the extension portion 211 may protrude beyond the rear handle 2, such that it may be possible to allow the operating doctor to visually observe a change in the sliding stroke externally, thereby enabling indirect control of the pulling force magnitude during the ligation process and helping to prevent an operational error.

In some embodiments, a ligature-cutting base 27 may be arranged in the rear handle 2, such that it may be possible to ensure that the ligature 4 may be rapidly cut by the ligature-cutting blade 25 under the preset pulling force. The ligature-cutting blade 25 may be opposite to the ligature-cutting base 27. The ligature 4 may be placed on the ligature-cutting base 27. A V-shaped notch/groove may be defined on the ligature-cutting base 27 and opposite to the ligature-cutting blade 25. When it is necessary to cut the ligature, the ligature-cutting blade 25 may be capable of rotating to abut against the V-shaped notch, and the ligature 4 may be subjected to greater tension at the V-shaped notch, such that the ligature 4 may be rapidly cut.

In some embodiments, the ligature-cutting blade 25 may be a hard ceramic blade, and the blade may be hard and sharp, thereby enabling rapid completion of the cutting process. In this way, it may be possible to reduce the risk of over-tightening the loop 41 and scratching cardiac tissue.

In some embodiments, the catheter assembly may include a heat-shrinkable sleeve 5, a flexible catheter 6, and a connecting tube 7. An end of the flexible catheter 6 may be connected to the front end of the front handle 1. A heat-shrinkable sleeve 5 may be covered on a connection part between the flexible catheter 6 and the front end of the front handle 1. An end of the connecting tube 7 may be connected to another end of the flexible catheter 6. Another end of the connecting tube 7 may be connected to the snare 3.

In some embodiments, the flexible catheter 6 may be made of flexible tubing material, which may offer excellent resistance to kinking, fracture, and plastic deformation, such that the flexible catheter 6 may be suitable for various bending scenarios, thereby significantly improving clinical efficiency. The connecting tube 7 may be a metal component, so as to provide sufficient strength at a front end of the handle assembly, thereby ensuring optimal retraction of the loop 41.

In some embodiments, the snare 3 may be designed with different diameter specifications according to different requirements. The whole snare 3 may be wrapped by a soft thermoplastic polyurethane (TPU) material, such it may be possible to protect a myocardial tissue and reduce accidental injury. A bone positioning component or a skeleton inside the snare 3 may be made of a nitinol memory alloy wire, so as to meet the usage environment or a channel with a narrow or curved condition.

In some embodiments, the snare 3 may be a flexible ring with built-in dual channels. The dual channels may be disposed externally and internally, and the loop 41 may be embedded in an inner channel of the dual channels.

In some embodiments, a radiopaque tungsten wire 31 may be embedded in an outer channel of the snare 3, and a radiopaque coil spring 71 may be disposed in the connection tube 7, so as to enhance imaging clarity and prevent an operational error. In this way, it may be possible to enable a position of the snare 3 to be clearly displayed on an imaging module via the radiopaque tungsten wire 31 and the radiopaque coil spring 71, thereby improving operability.

As described above, this is the specific embodiments of the present disclosure, but the protection scope of the present disclosure is not limited thereto. Any person skilled in the art may easily think of various equivalent modifications or substitutions within the technical scope disclosed in the present disclosure, and these modifications or substitutions should be covered within the protection scope of the present disclosure. Therefore, the protection scope of the present disclosure should be subject to the protection scope of the claims.

## Claims

1. An automatic ligature-cutting ligation device, comprising:
a handle assembly, comprising a front handle and a rear handle detachably connected to each other;
a catheter assembly, an end of the catheter assembly being connected to the front handle;
a snare, disposed at another end of the snare, wherein a slit is defined in an inner surface of the snare;
a ligature, penetrating the front handle and the catheter assembly, wherein an end of the ligature is connected to the rear handle, another end of the ligature has a loop capable of contracting in a forward direction and stopping in a reverse direction, and the loop is pre-mounted in the snare; and
a ligature-cutting assembly, disposed in the rear handle;
wherein in a case where the rear handle is separated from the front handle and pulled rearward, the ligature is driven to pull rearward, and the loop is driven to pull out of the snare from the slit to an inner side enclosed by the snare and contract in the forward direction; and
in a case where the rear handle is separated from the front handle and pulled rearward until a preset pulling force is reached, the ligature-cutting assembly is driven to move and cut the ligature.

2. The automatic ligature-cutting ligation device as claimed in claim 1, wherein an end of the front handle close to the rear handle is spirally interlocked with an end of the rear handle close to the front handle, and in a case where the rear handle rotates relative to the front handle, the rear handle is capable of being separated from or engaged with the front handle.

3. The automatic ligature-cutting ligation device as claimed in claim 1, wherein the ligature-cutting assembly comprises:
a sliding member, slidably mounted in the rear handle and capable of sliding in a direction close to the front handle or away from the front handle, wherein an end of the ligature is connected to the sliding member;
a connecting rod member, mounted in the rear handle, wherein an end of the connecting rod member is connected to the sliding member;
an elastic member, an end of the elastic member being connected to an inner side of the rear handle, and another end of the elastic member being connected to the sliding member; and
a ligature-cutting blade, connected to another end of the connecting rod member;
wherein in a case where the rear handle is pulled rearward, the ligature is configured to drive the sliding member to slide forward, the elastic member is driven to stretch, and the connecting rod member is simultaneously driven to rotate, such that the ligature-cutting blade is driven to rotate towards the ligature; and
in a case where the elastic member is stretched to the preset pulling force, the ligature is cut by the ligature-cutting blade.

4. The automatic ligature-cutting ligation device as claimed in claim 3, wherein the connecting rod member comprises a first connecting rod and a second connecting rod;
an end of the first connecting rod is rotatably connected to the sliding member, and another end of the first connecting rod is rotatably connected to an end of the second connecting rod; and
a rod arm of the second connecting rod is rotatably connected to an inner wall of the rear handle, and the ligature-cutting blade is disposed at another end of the second connecting rod.

5. The automatic ligature-cutting ligation device as claimed in claim 3, wherein a strip-shaped opening is defined on the rear handle, the sliding member is arranged with an extension portion, and the extension portion passes through the strip-shaped opening and protrudes beyond the rear handle; and
the extension portion is connected to another end of the elastic member.

6. The automatic ligature-cutting ligation device as claimed in claim 3, wherein the elastic member comprises one of a spiral spring and an elastic pull rope.

7. The automatic ligature-cutting ligation device as claimed in claim 3, wherein a ligature-cutting base is disposed in the rear handle, the ligature-cutting blade is opposite to the ligature-cutting base, and the ligature is placed on the ligature-cutting base; and
a V-shaped notch opposite to the ligature-cutting blade is defined on the ligature-cutting base.

8. The automatic ligature-cutting ligation device as claimed in claim 1, wherein the catheter assembly comprises a heat-shrinkable sleeve, a flexible catheter, and a connecting tube;
an end of the flexible catheter is connected to a front end of the front handle;
the heat-shrinkable sleeve is covered on a connection part between the flexible catheter and the front end of the front handle; and
an end of the connecting tube is connected to another end of the flexible catheter, and another end of the connecting tube is connected to the snare.

9. The automatic ligature-cutting ligation device as claimed in claim 1, wherein a bone positioning component inside the snare comprises a nitinol memory alloy wire.

10. The automatic ligature-cutting ligation device as claimed in claim 1, wherein a radiopaque tungsten wire is embedded in the snare, and a radiopaque coil spring is disposed in the connecting tube.
